# FASCICULE DE BREVET EUROPEEN

(11) **EP 2 438 025 B1**
(45) Date de publication et mention de la délivrance du brevet: **27.12.2017**
(21) Numéro de dépôt: 10734210.7
(22) Date de dépôt: 02.06.2010
(51) Int. Cl.: C03C 25/24, B01D 53/72, B01D 53/02, A61L 9/014, C09D 7/12, D04H 3/00, D04H 3/12

(54) **MAT DE FIBRES MINÉRALES RENFERMANT UN AGENT APTE À PIÉGER LE FORMALDÉHYDE ET PROCÉDÉS DE FABRICATION**
MINERALFASERMATTE MIT EINEM FORMALDEHYDFÄNGER UND HERSTELLUNGSVERFAHREN DAFÜR
MINERAL FIBER MAT CONTAINING A FORMALDEHYDE-TRAPPING AGENT, AND METHODS FOR MANUFACTURING SAME

(30) Priorité: 03.06.2009 FR 0953654
(43) Date de publication de la demande: 11.04.2012
(73) Titulaire: Saint-Gobain Adfors, 73000 Chambéry (FR)
(72) Inventeur: BLANCHARD, Benjamin, F-95150 Taverny (FR); CHUDA, Katarzyna, F-75016 Paris (FR); JAFFRENNOU, Boris, F-75019 Paris (FR)
(74) Mandataire: Saint-Gobain Recherche
(86) Numéro de dépôt international: PCT/FR2010/051073
(87) Numéro de publication internationale: WO 2010/139897

(56) Documents cités:
- EP-A- 1 424 432
- EP-A- 1 905 560
- WO-A1-2010/040963
- WO-A2-2010/070248
- JP-A- 2001 178 805
- US-A- 5 160 503
- US-A- 5 160 679
- US-A- 5 194 674
- US-A- 5 795 933
- US-A1- 2008 038 971
- US-A1- 2008 135 060

## Description

L'invention se rapporte à un mat de fibres minérales qui renferme un agent apte à piéger le formaldéhyde et aux procédés permettant sa fabrication.

Des matériaux composites très divers sont utilisés dans le domaine de la construction et de l'aménagement de bâtiments d'habitation et de bureau, ainsi que de véhicules de transport. Certains de ces matériaux tels que les isolants acoustiques et/ou thermiques, les panneaux de bois, les éléments de mobilier et de décoration, utilisent des adhésifs, des peintures et des vernis contenant des résines à base de formaldéhyde. La proportion de formaldéhyde dans ces matériaux est déjà très faible.

Cependant, la réglementation en matière de protection contre les émissions indésirables de produits tels que le formaldéhyde qui peuvent présenter un risque pour la santé des individus devient plus stricte et impose de réduire encore la quantité de formaldéhyde libre ou susceptible d'être émis par les matériaux au cours du temps.

Des moyens pour réduire la teneur en formaldéhyde à l'intérieur de bâtiments sont connus.

Il a été proposé d'inclure des particules d'oxyde de titane photocatalytique dans une peinture ou un matériau en plâtre (US-A-2005/0226761), un papier ou un matériau textile, plastique ou en bois (EP-A-1 437 397).

Il est aussi connu d'utiliser un hydrazide dans un matériau de construction à base de plâtre ou de ciment (US-A-2004/0101695 et JP-A-2004115340).

Il est encore connu d'utiliser un carbodihydrazide dans un panneau de fibres de bois pour capturer et décomposer le formaldéhyde et l'acétaldéhyde (EP 1 905 560). JP 2001 178805 décrit l'utilisation d'un agent piégeur de formaldéhyde contenant des groupes hydrazides pour un mat de fibres minérales. La présente invention a pour but de réduire la quantité de formaldéhyde présent à l'intérieur des bâtiments, notamment d'habitation, et de véhicules de transport.

Pour atteindre ce but, la présente invention propose un mat à base de fils minéraux selon la revendication 1. Un autre objet de l'invention concerne les procédés qui permettent de fabriquer ledit mat à base de fils minéraux. Par « composé apte à réagir avec le formaldéhyde » on entend un composé organique qui se lie au formaldéhyde par liaison covalente. Le composé apte à réagir avec le formaldéhyde est les composés à méthylène(s) actif(s), répondant aux formules suivantes : 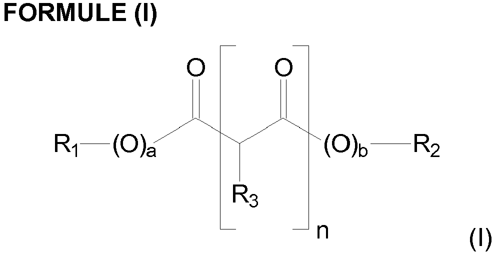
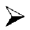 dans laquelle :
   - R₁ et R₂, identiques ou différents, représentent un atome d'hydrogène, un radical alkyle en C₁-C₂₀, de préférence C₁-C₆, un radical amino ou un radical de formule 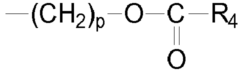 dans laquelle R₄ représente un radical 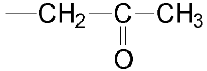 ou 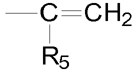 où R₅ = H ou -CH₃ et p est un nombre entier variant de 1 à 6
   - R₃ représente un atome d'hydrogène, un radical alkyle en C₁-C₁₀, un radical phényle ou un atome d'halogène
   - a est égal à 0 ou 1
   - b est égal à 0 ou 1
   - n est égal à 1 ou 2

Les composés de formule (I) préférés sont :
- la 2,4-pentanedione :
   R₁ = -CH₃ ; R₂ = -CH₃ ; R₃ = H ; a = 0 ; b = 0 ; n = 1
- la 2,4-hexanedione :
   R₁ = -CH₂-CH₃ ; R₂ = -CH₃ ; R₃ = H ; a = 0 ; b = 0 ; n = 1
- la 3,5-heptanedione
   R₁ = -CH₂-CH₃ ; R₂ = -CH₂-CH₃ ; R₃ = H ; a = 0 ; b = 0 ; n = 1
- la 2,4-octanedione :
   R₁ = -CH₃ ; R₂ = -(CH₂)₃-CH₃ ; R₃ = H ; a = 0 ; b = 0 ; n = 1
- l'acétoacétamide :
   R₁ = -CH₃ ; R₂ = -NH₂ ; R₃ = H ; a = 0 ; b = 0 ; n = 1
- l'acide acétoacétique :
   R₁ = -CH₃ ; R₂ = H ; R₃ = H ; a = 0 ; b = 1 ; n = 1
- le méthylacétoacétate :
   R₁ = -CH₃ ; R₂ = -CH₃ ; R₃ = H ; a = 0 ; b = 1 ; n = 1
- l'éthylacétoacétate :
   R₁ = -CH₃ ; R₂ = -CH₂-CH₃ ; R₃ = H ; a = 0 ; b = 1 ; n = 1
- le n-propylacétoacétate :
   R₁ = -CH₃ ; R₂ = -(CH₂)₂-CH₃ ; R₃ = H ; a = 0 ; b = 1 ; n = 1
- l'iso-propylacétoacétate :
   R₁ = -CH₃ ; R₂ = -CH(CH₃)₂ ; R₃ = H ; a = 0 ; b = 1 ; n = 1
- l'iso-butylacétoacétate :
   R₁ = -CH₃ ; R₂ = -CH₂-CH(CH₃)₂ R₃ = H ; a = 0 ; b = 1 ; n = 1
- le t-butylacétoacétate :
   R₁ = -CH₃ ; R₂ = -C(CH₃)₃ ; R₃ = H ; a = 0 ; b = 1 ; n = 1
- le n-hexylacétoacétate :
   R₁ = -CH₃ ; R₂ = -(CH₂)₅-CH₃ R₃ = H ; a = 0 ; b = 1 ; n = 1
- le malonamide :
   R₁ = -NH₂ ; R₂ = -NH₂ ; R₃ = H ; a = 0 ; b = 0 ; n = 1
- l'acide malonique :
   R₁ = H; R₂ = H; R₃ = H ; a = 1 ; b = 1 ; n = 1
- le diméthylmalonate :
   R₁ = -CH₃ ; R₂ = -CH₃ ; R₃ = H; a = 1 ; b = 1 ; n = 1
- le diéthylmalonate :
   R₁ = -CH₂-CH₃ ; R₂ = -CH₂-CH₃ ; R₃ = H; a = 1; b = 1; n = 1
- le di-n-propylmalonate :
   R₁ = -(CH₂)₂-CH₃ ; R₂ = -(CH₂)₂-CH₃ ; R₃ = H ; a = 1 ; b = 1 ; n = 1
- le di-iso-propylmalonate :
   R₁ = -CH(CH₃)₂ ; R₂ = -CH(CH₃)2 ; R₃ = H ; a = 1 ; b = 1 ; n = 1
- le di-n-butylmalonate :
   R₁ = -(CH₂)₃-CH₃ ; R₂ = -(CH₂)₃-CH3 ; R₃ = H ; a = 1 ; b = 1 ; n = 1
- l'acide acétonedicarboxylique :
   R₁ = H; R₂ = H; R₃ = H; a = 1 ; b = 1 ; n = 2
- le diméthylacétonedicarboxylate :
   R₁ = -CH₃ ; R₂ = -CH₃ ; R₃ = H ; a = 1 ; b = 1 ; n = 2
- le 1,4-butanedioldiacétate
   R₁ = -CH₃ ; R₂ = -(CH₂)₄-O-CO-CH₂-CO-CH₃ ; R₃ = H ; a = 0 ; b = 1 ; n = 1
- le 1,6-hexanedioldiacétate
   R₁ = -CH₃ ; R₂ = -(CH₂)₆-O-CO-CH₂-CO-CH₃ ; R₃ = H ; a = 0 ; b = 1 ; n = 1
- le méthacryloxyéthylacétoacétate
   R₁ = -CH₃ ; R₂ = -(CH₂)₂-O-CO-C(CH₃)=CH₂ ; R₃ = H ; a = 0 ; b = 1 ; n = 1.

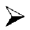 **FORMULE (II)** R₆-CHR₇-C≡N (II)
dans laquelle
- R₆ représente un radical cyano ou un radical 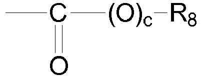 dans lequel :
   - R₈ représente un atome d'hydrogène, un radical alkyle en C₁-C₂₀, de préférence C₁-C₆, ou un radical amino
   - c est égal à 0 ou 1
- R₇ représente un atome d'hydrogène, un radical alkyle en C₁-C₁₀,
   un radical phényle ou un atome d'halogène.

Les composés de formule (II) préférés sont :
- le 2-méthylcyanoacétate :
   R₆ = -CO-O-CH₃ ; R₇ = H
- le 2-éthylcyanoacétate :
   R₆ = -CO-O-CH₂-CH₃ ; R₇ = H
- le 2-n-propylcyanoacétate :
   R₆ = -CO-O-(CH₂)₂-CH₃ ; R₇ = H
- le 2-iso-propylcyanoacétate :
   R₆ = -CO-O-CH(CH₃)₂ ; R₇ = H
- le 2-n-butylcyanoacétate :
   R₆ = -CO-O-(CH₂)₃CH₃ ; R₇ = H
- le 2-iso-butylcyanoacétate :
   R₆ = -CO-O-CH₂-CH(CH₃)₂ ; R₇ = H
- le 2-ter-butylcyanoacétate :
   R₆ = -CO-O-C(CH₃)₃ ; R₇ = H
- le 2-cyanoacétamide :
   R₆ = -CO-NH₂ ; R₅ = H
- le propanedinitrile :
   R₆ = -CΞN ; R₅ = H 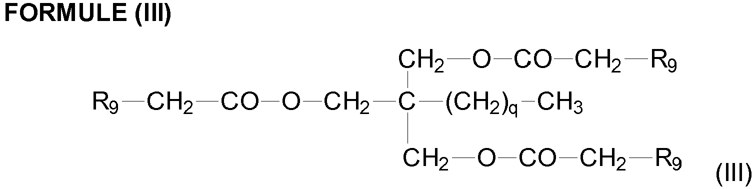
      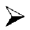 dans laquelle
      - R₉ représente un radical -C≡N ou -CO-CH₃
      - q est un nombre entier variant de 1 à 4.

Les composés de formule (III) préférés sont :
- le triméthylolpropane triacétoacétate :
   R₉ = -CO-CH₃ ; q = 1
- le triméthylolpropane tricyanoacétate :
   R₉ =-CΞN ; q = 1 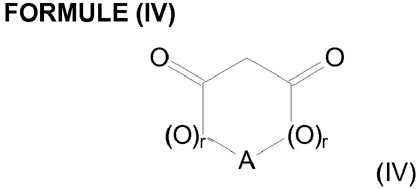
      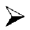 dans laquelle
         - A représente un radical -(CH₂)₃- ou -C(CH₃)₂-
         - r est égal à 0 ou 1

Les composés de formule (IV) préférés sont :
- la 1,3-cyclohexanedione :
   A = -(CH₂)₃ ; r = 0
- l'acide de Meldrum :
   A = -C(CH₃)₂- ; r = 1.
La quantité d'agent apte à piéger le formaldéhyde à utiliser peut varier dans une large mesure, par exemple de 0,1 à 500 g/m² de mat, de préférence de 0,5 à 100 g/m² et avantageusement de 1 à 50 g/m².

Le cas échéant, l'agent apte à piéger le formaldéhyde peut être utilisé en combinaison avec au moins un matériau poreux qui adsorbe les composés organiques volatils, notamment les composés aromatiques tels que le xylène, le benzène et le toluène.

Ce matériau poreux se présente sous la forme de particules ayant une dimension qui varie de 10 nm à 100 µm, de préférence 500 nm à 50 µm, et avantageusement 1 à 10 µm. De préférence, les particules présentent une surface spécifique qui varie de 1 à 5000 m²/g, avantageusement 5 à 2000 m²/g, notamment supérieure à 100 m²/g et un diamètre de pore moyen variant de 1 à 50 nm, de préférence 1 à 20 nm.

Le matériau poreux peut être :
- de la silice, microporeuse ou mésoporeuse, précipitée ou non, pyrogénée ou non, pouvant contenir des nanoparticules de complexes métalliques d'oxydes, d'hydroxydes, d'hydrates ou de polyoxométallates,
- un noir de carbone pouvant contenir des nanoparticules de complexes métalliques d'oxydes, d'hydroxydes, d'hydrates ou de polyoxométallates,
- un oxyde d'aluminium activé et de permanganate de potassium,
- une zéolithe, naturelle ou synthétique,
- un polymère, par exemple un polyamide.

Le mat conforme à l'invention est à base de fibres minérales et peut éventuellement contenir des fibres constituées d'une matière organique par exemple une oléfine telle que le polyéthylène et le polypropylène ou un polytéréphtalate d'alkylène tel que le polytéréphtalate d'éthylène.

Par « fibres », on entend aussi bien des filaments que des fils composés d'une multitude de filaments liés ensemble, notamment par un ensimage, et des assemblages de tels fils.

La matière minérale constituant les fibres précitées est de préférence un verre ou une roche, notamment un basalte.

Ainsi, selon un premier mode de réalisation, le mat de fibres minérales est composé des filaments minéraux discontinus de longueur pouvant atteindre 150 mm, de préférence variant de 20 et 100 mm et avantageusement de 50 à 70 mm, et ayant un diamètre qui peut varier dans une large mesure, par exemple de 5 à 30 µm.

Selon un deuxième mode de réalisation, le mat de fibres minérales est composé de fils minéraux.

Les fils minéraux peuvent être des fils composés d'une multitude de filaments minéraux (ou fils de base) ou des assemblages de ces fils de base en stratifils (« rovings » en anglais), des fils « comêlés » constitués de filaments minéraux et de filaments de la matière organique précitée qui sont intimement mélangés, ou des fils mixtes comprenant au moins un fil composé d'une multitude de filaments minéraux et au moins un fil composé d'une multitude de filaments de la matière organique thermoplastique précitée.

Les fils précités peuvent être des fils sans torsion ou des fils retordus (ou fils textiles), de préférence sans torsion.

Les fils minéraux, notamment de verre, sont coupés à une longueur pouvant aller jusqu'à 100 mm, de préférence variant de 6 à 30 mm, avantageusement de 8 à 20 mm et mieux encore de 10 à 18 mm.

Le diamètre des filaments de verre constituant les fils peut varier dans une large mesure, par exemple 5 à 30 µm. De la même manière, de larges variations peuvent survenir dans la masse linéique du fil qui peut aller de 34 à 1500 tex.

Le verre entrant dans la constitution des filaments peut être de tout type, par exemple E, C, R, AR (alcali-résistant). On préfère le verre E.

Le mat de fibres minérales selon l'un ou l'autre mode de réalisation présente une masse surfacique qui varie de 10 à 1100 g/m², de préférence 20 à 300 g/m².

De manière classique, le mat de fibres minérales contient un liant qui lie lesdites fibres et lui confère des propriétés mécaniques adaptées à l'usage désiré, notamment une rigidité suffisante pour pouvoir être manipulé facilement.

Le liant comprend généralement au moins un polymère apte à lier les fibres minérales. Ce polymère peut être un polymère thermoplastique, par exemple le styrène-acrylonitrile, l'acrylonitrile-butadiène-styrène, le (tri)acétate de cellulose, le polystèrene expansé, une polyoléfine telle que le polyéthylène et le polypropylène, un poly(méth)acrylate, un polyacétate de vinyle ou un polyoxyméthylène ; un polymère thermodurcissable, par exemple un polyester insaturé, un epoxyde, une résine phénolique telle qu'une novolaque ou un résol, notamment ayant un taux d'aldéhyde(s) libre(s) inférieur à 0,05 %, un polyimide, un polyuréthane, un phénoplaste ou un biopolymère par exemple un polysaccharide ou une protéine ; un polymère élastomère, par exemple un polymère fluoré, notamment à base de fluorure de vinylidène, le néoprène, un polyacrylique, un polybutadiène, un polyéther amide, un silicone, un caoutchouc naturel ou styrène-butadiène (SBR), ou un biopolymère, par exemple un polysaccharide ou une protéine.

Le liant représente généralement 1 à 1000 % en poids du mat de fibres minérales, de préférence 5 à 350 %, et avantageusement 10 à 100 %.

Les procédés de fabrication du mat de fibres minérales et du mat de fils minéraux constituent d'autres objets de la présente invention.

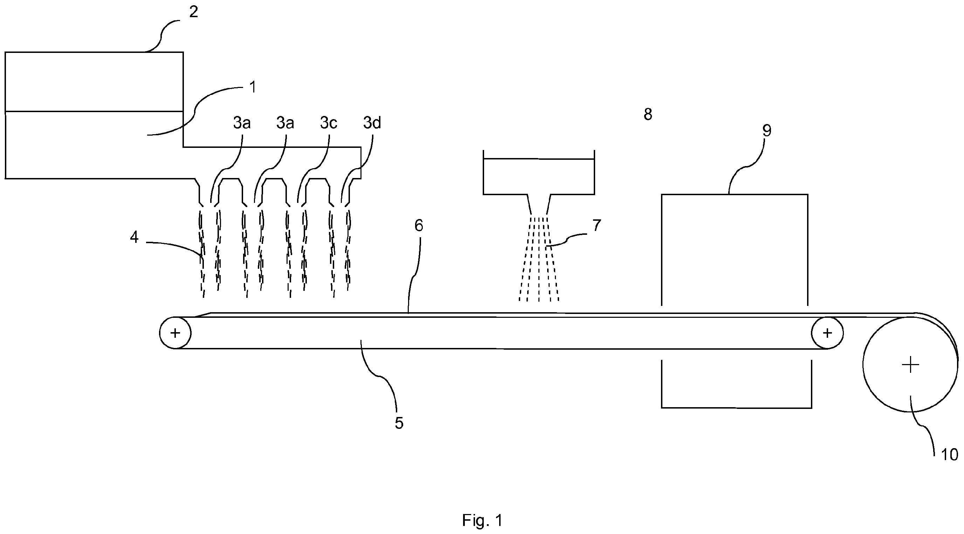

La figure 1 est une vue schématique d'une installation conventionnelle permettant la fabrication d'un mat de filaments minéraux selon un procédé opérant par « voie sèche ».

De la matière minérale fondue (1) contenue dans un four (2) est dirigée vers un ensemble de plusieurs filières (3 a-d) à partir desquelles des filaments (4) s'écoulent par gravité et sont étirés par un fluide gazeux. Les filaments (4) sont récoltés sur un convoyeur (5) se déplaçant dans le sens indiqué par la flèche où ils s'entremêlent en formant un mat (6).

Un liant (7) est appliqué sur le mat (6) à l'aide d'un dispositif (8) opérant par pulvérisation, puis le mat entre dans un dispositif de séchage (9) à air chaud dont la température est adaptée pour éliminer l'eau et éventuellement réticuler le liant.

D'autres dispositifs de séchage peuvent être utilisés, par exemple un dispositif opérant par rayonnement infrarouge ou comprenant un ou plusieurs rouleaux chauffants.

A la sortie du dispositif de séchage (9), le mat (6) est collecté sous la forme d'un enroulement (10).

Conformément à l'invention, on ajoute à cette installation conventionnelle une étape de traitement avec un agent apte à piéger le formaldéhyde.

Selon un premier mode de réalisation, on introduit l'agent apte à piéger le formaldéhyde dans le liant (7). Ce mode de réalisation est préféré car il ne requiert aucun dispositif supplémentaire pour l'application de l'agent apte à piéger le formaldéhyde, ce qui est avantageux d'un point de vue économique.

Selon un deuxième mode de réalisation, on applique l'agent apte à piéger le formaldéhyde après que le liant (7) est déposé sur le mat (6) et avant que celui-ci entre dans le dispositif de séchage (9).

L'application dudit agent peut être effectuée par tout moyen connu, de préférence à l'aide d'un dispositif opérant par pulvérisation.

Par exemple, ce dispositif peut être constitué d'une pluralité de buses de pulvérisation alimentées par une solution aqueuse de l'agent apte à piéger le formaldéhyde qui génèrent des jets divergents qui s'interpénètrent peu avant d'arriver au contact de la face supérieure du mat (6).

L'application de l'agent apte à piéger le formaldéhyde peut encore se faire en reprise, dans une étape postérieure à la collecte du mat sous forme d'enroulement (10), par exemple en faisant passer le mat dans un bain contenant ledit agent. Cette manière de procéder est cependant plus onéreuse que les précédentes car elle impose une étape supplémentaire de déroulement du mat et des moyens spécifiques pour l'application de l'agent apte à piéger le formaldéhyde sous forme de solution aqueuse et pour l'élimination de l'eau.

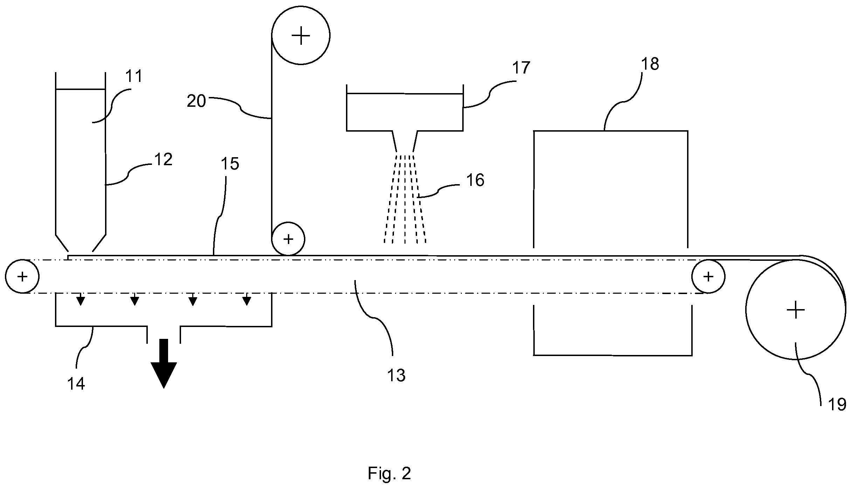

La figure 2 est une vue schématique d'une installation conventionnelle permettant la fabrication d'un mat de fils minéraux selon un procédé opérant par « voie humide ».

Une dispersion de fils minéraux coupés dans de l'eau (11) est déposée au moyen d'une tête de formage (12) sur un convoyeur (13) muni de perforations. Au contact du convoyeur (13), l'eau contenue dans la dispersion (11) est extraite par un caisson d'aspiration (14). Sur le convoyeur, les fils minéraux coupés forment un mat (15) sur lequel est déposé un liant (16) au moyen d'un dispositif de pulvérisation (17). Le mat (15) entre ensuite dans un dispositif de séchage (18) à air chaud dont la température est adaptée pour éliminer l'eau résiduelle et éventuellement obtenir la réticulation du liant.

De même que dans le procédé opérant par la voie sèche, le dispositif de séchage (18) peut être remplacé par un dispositif opérant par rayonnement infrarouge ou comprenant un ou plusieurs rouleaux chauffants.

Le mat (15) est ensuite collecté sous la forme d'un enroulement (19).

Le mat peut, le cas échéant, être renforcé par des fibres continues (20) disposées dans le sens d'avancement du mat et réparties sur tout ou partie de la largeur du mat. Ces fibres (20) sont généralement déposées sur le mat (15) avant l'application du liant (16).

Les fibres (20) peuvent être des fibres synthétiques ou naturelles.

A titre d'exemples de fibres synthétiques, on peut citer les fibres inorganiques, notamment de verre ou de roche telle que le basalte, et les fibres organiques, notamment de polyamide, de polyester ou d'une polyoléfine telle que le polyéthylène et le polypropylène. Le verre est préféré.

A titre d'exemples de fibres naturelles, on peut citer les fibres végétales, notamment de coton, de noix de coco, de sisal, de chanvre ou de lin, et les fibres animales notamment la soie ou la laine.

Conformément à l'invention, on ajoute à cette installation conventionnelle une étape de traitement avec un agent apte à piéger le formaldéhyde.

L'étape de traitement peut être effectuée dans les conditions déjà exposées pour le procédé par voie sèche, c'est-à-dire en introduisant l'agent apte à piéger le formaldéhyde dans le liant (16), en appliquant celui-ci après que le liant a été déposé sur le mat (15) et avant que celui-ci entre dans le dispositif de séchage (18), ou encore en reprise, dans une étape postérieure a la collecte du mat sous forme d'enroulement (19).

Bien que l'invention soit décrite relativement au formaldéhyde, il est vraisemblable que les agents précités aptes à piéger ce composé sont également aptes à piéger l'acétaldéhyde.

Le mat de fibres minérales conforme à la présente invention peut être utilisé dans de nombreuses applications, par exemple en tant que revêtement, à peindre ou non, pouvant être appliqué sur les murs et/ou les plafonds, revêtement de surface ou de jointement de panneaux de plâtre ou de ciment, revêtement de surface de produits d'isolation thermique et/ou phonique tels qu'une laine minérale ou une mousse destinée plus particulièrement à l'isolation des toitures ou pour la réalisation de revêtement de sol tel qu'une moquette ou un matériau vinylique.

Les exemples qui suivent permettent d'illustrer l'invention sans toutefois la limiter.

### EXEMPLES 1 ET 2

### a) fabrication du mat de verre

On prépare deux litres d'une solution aqueuse contenant 250 g d'hydroxyéthylcellulose (épaississant ; commercialisé sous la référence Natrosol® 250 HHR par la société Hercules) et 0,3 g d'un complexe octadécylamine-octadécylguanidine éthoxylé (agent tensioactif ; commercialisé sous la référence Aerosol® C-61 par la société Cytec ; teneur en matières sèches : 70 %).

A la solution précitée, on ajoute 2,54 g de fils de verre E (... tex ; diamètre des filaments : 13 µm) coupés (longueur 8 mm).

La suspension de fils de verre obtenue est utilisée dans un dispositif permettant la réalisation d'un mat. Le dispositif comprend une grille surmontée d'un caisson étanche aux liquides et un caisson d'aspiration situé sous la grille.

La suspension est déposée dans le caisson étanche, homogénéisée par agitation vigoureuse, puis le caisson d'aspiration est mis en action de manière à éliminer l'eau. On récupère sur la grille un mat de fibres de verre de dimensions 30 cm x 30 cm ayant une masse surfacique égale à 28,2 g/m².

Le mat est immergé pendant 1 minute dans une solution aqueuse d'un liant contenant une résine acrylique (Acrodur® 950L commercialisé par BASF) et l'agent apte à piéger le formaldéhyde, à savoir l'acétoacétamide (exemple 1) ou le dihydrazide d'acide adipique (exemple de référence 2).

L'excès de liant dans le mat est éliminé par aspiration, puis le mat est chauffé à 210°C pendant 1 minute afin de le consolider.

Au final, le mat renferme 5 g/m² de résine acrylique et 2,5 g/m² d'agent apte à piéger le formaldéhyde. Il présente une bonne stabilité dimensionnelle et une bonne tenue mécanique.

### b) aptitude à piéger le formaldéhyde

Un échantillon du mat est placé dans un dispositif conforme à la norme ISO 16000-9, modifié en ce que le débit de ventilation spécifique est égal à 1,48 m³/(m².h) et le taux de charge est égal à 0,27 m²/m³.
1 - dans un premier temps, la chambre de test du dispositif est alimentée avec un flux d'air continu contenant de l'ordre de 50 µg/m³ de formaldéhyde pendant 7 jours. On mesure la quantité de formaldéhyde dans l'air entrant et sortant de la chambre sur une durée de 7 jours, et on calcule la réduction de la quantité de formaldéhyde par unité de volume d'air.
Le formaldéhyde est mesuré par chromatographie en phase liquide (HPLC) dans les conditions de la norme ISO 16000-3.
Dans le tableau 1, on indique la réduction de la quantité de formaldéhyde opérée avec le mat contenant l'agent apte à piéger le formaldéhyde (exemples 1 et 2) par comparaison avec un mat fabriqué dans les conditions décrites au paragraphe a) ne contenant pas d'agent apte à piéger le formaldéhyde (Référence).

**Tableau 1**

| Réduction du formaldéhyde (µg/m³) | Ex. 1 | Ex. 2 | Réf. |
|---|---|---|---|
| 2 jours | 7 | 9 | 4 |
| 3 jours | 8 | 11 | 2 |
| 7 jours | 8 | 10 | 1 |

2 - dans un deuxième temps, on alimente la chambre pendant 7 jours avec de l'air ne contenant pas de formaldéhyde et on mesure la quantité de formaldéhyde présent dans l'air à la sortie de la chambre.

Le formaldéhyde est mesuré dans les mêmes conditions qu'au paragraphe 1.

La quantité de formaldéhyde émise par le mat selon les exemples 1 et 2 est équivalente à celle que l'on mesure lorsque la chambre ne contient aucun mat. On peut en conclure que le formaldéhyde est lié à l'agent apte à piéger le formaldéhyde de manière forte et durable.

## Revendications

1. Mat à base de fils minéraux liés par un liant, lesdits fils comprenant une multitude de filaments minéraux, et lesdits fils étant coupés à une longueur pouvant aller jusqu'à 100 mm, **caractérisé en ce qu'**il renferme un agent apte à piéger le formaldéhyde choisis parmi les composés à méthylène(s) actif(s) répondant à l'une des formules (I) à (IV) suivantes :
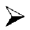 **FORMULE (I)** 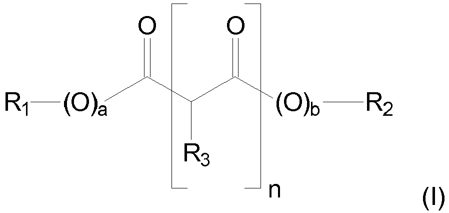 dans laquelle :
- R₁ et R₂, identiques ou différents, représentent un atome d'hydrogène, un radical alkyle en C₁-C₂₀, de préférence C₁-C₆, un radical amino ou un radical de formule 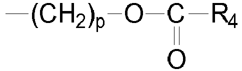 dans laquelle R₄ représente un radical 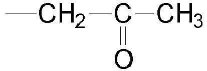 ou 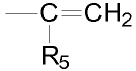 où R₅ = H ou -CH₃
et p est un nombre entier variant de 1 à 6
- R₃ représente un atome d'hydrogène, un radical alkyle en C₁-C₁₀, un radical phényle ou un atome d'halogène
- a est égal à 0 ou 1
- b est égal à 0 ou 1
- n est égal à 1 ou 2
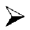 **FORMULE (II)**
R6-CHR₇-C≡N (II)
dans laquelle
- R₆ représente un radical cyano ou un radical 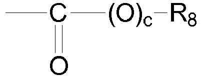 dans lequel :
- R₈ représente un atome d'hydrogène, un radical alkyle en C₁-C₂₀, de préférence C₁-C₆, ou un radical amino
- c est égal à 0 ou 1
- R₇ représente un atome d'hydrogène, un radical alkyle en C₁-C₁₀,
un radical phényle ou un atome d'halogène
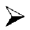 **FORMULE (III)** 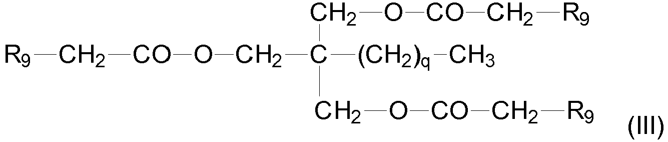 dans laquelle
- R₉ représente un radical -C≡N ou -CO-CH₃
- q est un nombre entier variant de 1 à 4
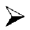 **FORMULE (IV)** 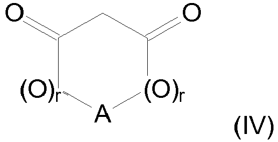 dans laquelle
- A représente un radical -(CH₂)₃- ou -C(CH₃)₂-
- r est égal à 0 ou 1

2. Mat selon la revendication 1, **caractérisé en ce que** le composé de formule (I) est la 2,4-pentanedione, la 2,4-hexanedione, la 3,5-heptanedione, la 2,4-octanedione, l'acétoacétamide, l'acide acétoacétique, le méthylacétoacétate, l'éthylacétoacétate, le n-propylacétoacétate, l'iso-propylacétoacétate, l'iso-butylacétoacétate, le t-butylacétoacétate, le n-hexylacétoacétate, le malonamide, l'acide malonique, le diméthylmalonate, le diéthylmalonate, le di-n-propylmalonate, le di-iso-propylmalonate, le di-n-butylmalonate , l'acide acétonedicarboxylique et le diméthylacétonedicarboxylate.

3. Mat selon la revendication 1, **caractérisé en ce que** le composé de formule (II) est le 2-méthylcyanoacétate, le 2-éthylcyanoacétate, le 2-n-propylcyanoacétate, le 2-iso-propylcyanoacétate, le 2-n-butylcyanoacétate, le 2-iso-butylcyanoacétate, le 2-ter-butylcyanoacétate, le 2-cyanoacétamide et le propanedinitrile.

4. Mat selon la revendication 1, **caractérisé en ce que** le composé de formule (III) est le triméthylolpropane triacétoacétate et le triméthylolpropane tricyanoacétate.

5. Mat selon la revendication 1, **caractérisé en ce que** le composé de formule (IV) est la 1,3-cyclohexanedione et l'acide de Meldrum.

6. Mat selon l'une des revendications 1 à 5, **caractérisé en ce que** la teneur en composé apte à piéger le formaldéhyde varie de 0,1 à 500 g/m², de préférence de 0,5 à 100 g/m² et avantageusement de 1 à 50 g/m².

7. Mat selon l'une des revendications 1 à 6, **caractérisé en ce que** les fils minéraux sont des fils composés d'une multitude de filaments minéraux (ou fils de base) ou des assemblages de ces fils de base en stratifils, des fils « comêlés » constitués de filaments minéraux et de filaments de matière organique qui sont intimement mélangés, ou des fils mixtes comprenant au moins un fil composé d'une multitude de filaments minéraux et au moins un fil composé d'une multitude de filaments de matière organique thermoplastique.

8. Mat selon la revendication 7, **caractérisé en ce que** les filaments ou les fils sont en verre, de préférence en verre E.

9. Mat selon l'une des revendications 1 à 8, **caractérisé en ce qu'**il présente une masse surfacique variant de 10 à 1100 g/m², de préférence 20 à 300 g/m².

10. Mat selon l'une des revendications 1 à 9, **caractérisé en ce que** les fils sont sans torsion.

11. Procédé de fabrication du mat selon l'une des revendications 1 à 10, dans lequel on dépose une dispersion(11) de fils minéraux coupés dans de l'eau au moyen d'une tête de formage (12) sur un convoyeur (13) muni de perforations, on extrait l'eau au moyen d'un caisson d'aspiration (14), on récolte lesdits fils sous la forme d'un mat (15), on applique un liant (16) sur ledit mat, on fait passer ledit mat dans un dispositif de séchage (18) et on le collecte, **caractérisé en ce qu'**il comprend une étape de traitement avec un agent apte à piéger le formaldéhyde choisi parmi les composés à méthylène(s) actif(s) de formule (I) à (IV) précitées.

12. Procédé selon la revendication 11, **caractérisé en ce que** le traitement consiste à introduire ledit agent dans le liant (16).

13. Procédé selon la revendication 11, **caractérisé en ce que** le traitement consiste à appliquer ledit agent après que le liant (16) est déposé sur le mat et avant que le mat entre dans le dispositif de séchage (18).

14. Procédé selon la revendication 13, **caractérisé en ce que** l'application est effectuée par pulvérisation d'une solution aqueuse dudit agent.

## Patentansprüche

1. Matte auf Basis von mineralischen Fäden, die durch ein Bindemittel gebunden sind, wobei die Fäden eine Vielzahl von Mineralfilamenten umfassen und die Fäden auf eine Länge von bis zu 100 mm geschnitten sind, **dadurch gekennzeichnet, dass** die Matte einen Formaldehydfänger enthält, der aus den aktiven Methylenverbindungen entsprechend einer der folgenden Formeln (I) bis (IV) ausgewählt ist:
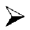 **FORMEL (I)** 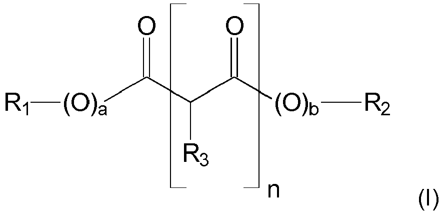 wobei :
- R₁ und R₂, identisch oder verschieden, für ein Wasserstoffatom, einen C₁-C₂₀-Alkylrest, bevorzugt C₁-C₆, ein Aminorest oder einen Rest der Formel 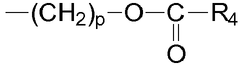 steht, wobei R₄ für einen Rest 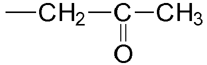 oder 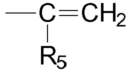 steht, wobei R₅ = H oder -CH₃ ist
und p eine ganze Zahl von 1 bis 6 ist
- R₃ für ein Wasserstoffatom, einen C₁-C₁₀-Alkylrest, einen Phenylrest oder ein Halogenatom steht,
- a gleich 0 oder 1 ist
- b gleich 0 oder 1 ist
- n gleich 1 oder 2 ist
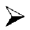 **FORMEL (II)**
R₆-CHR₇-C≡N (II)
wobei
- R₆ für einen Cyanorest oder einen Rest 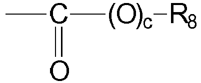 steht, wobei :
- R₈ für ein Wasserstoffatom, einen C₁-C₂₀-Alkylrest, bevorzugt C₁-C₆, oder einen Aminorest steht
- c gleich 0 oder 1 ist
- R₇ für ein Wasserstoffatom, einen C₁-C₁₀-Alkylrest, einen Phenylrest oder ein Halogenatom steht
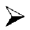 **FORMEL (III)** 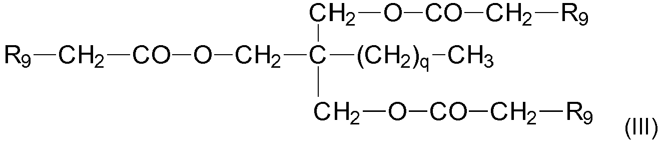 wobei
- R₉ für einen Rest -C≡N oder -CO-CH₃ steht
- q eine ganze Zahl von 1 bis 4 ist
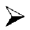 **FORMEL (IV)** 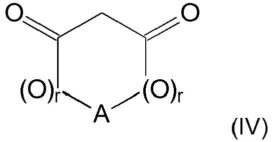 wobei
- A für einen Rest -(CH₂)₃- oder -C(CH₃)₂- steht
- r gleich 0 oder 1 ist

2. Matte nach Anspruch 1, **dadurch gekennzeichnet, dass** die Verbindung der Formel (I) 2,4-Pentandion, 2,4-Hexandion, 3,5-Heptandion, 2,4-Octandion, Acetacetamid, Acetessigsäure, Methylacetacetat, Ethylacetacetat, n-Propylacetacetat, Isopropylacetacetat, Isobutylacetacetat, t-Butylacetacetat, n-Hexylacetacetat, Malonamid, Malonsäure, Dimethylmalonat, Diethylmalonat, Di-n-propylmalonat, Diisopropylmalonat, Di-n-butylmalonat , Acetondicarbonsäure und Dimethylacetondicarbonsäure ist.

3. Matte nach Anspruch 1, **dadurch gekennzeichnet, dass** die Verbindung der Formel (II) 2-Methylcyanoacetat, 2-Ethylcyanoacetat, 2-n-Propylcyanoacetat, 2-Isopropylcyanoacetat, 2-n-Butylcyanoacetat, 2-Isobutylcyanoacetat, 2-ter-Butylcyanoacetat, 2-Cyanacetamid und Propandinitril ist.

4. Matte nach Anspruch 1, **dadurch gekennzeichnet, dass** die Verbindung der Formel (III) Trimethylolpropantriacetacetat und Trimethylolpropantricyanoacetat ist.

5. Matte nach Anspruch 1, **dadurch gekennzeichnet, dass** die Verbindung der Formel (IV) 1,3-Cyclohexandion und Meldrumsäure ist.

6. Matte nach einem der Ansprüche 1 bis 5, **dadurch gekennzeichnet, dass** der Gehalt an Formaldehydfängerverbindung zwischen 0,1 und 500 g/m², bevorzugt zwischen 0,5 und 100 g/m² und vorteilhafter zwischen 1 und 50 g/m² liegt.

7. Matte nach einem der Ansprüche 1 bis 6, **dadurch gekennzeichnet, dass** die mineralischen Fäden Fäden sind, die zusammengesetzt sind aus einer Vielzahl von Mineralfilamenten (oder Grundfäden) oder Anordnungen dieser Grundfäden als Rovings, aus « miteinander vermischten» Fäden, die aus innig vermischten Mineralfilamenten und Filamenten aus organischem Material bestehen, oder aus gemischten Fäden, die mindestens einen Faden umfassen, der aus einer Vielzahl von Mineralfilamenten zusammengesetzt ist, und mindestens einen Faden, der aus einer Vielzahl von Filamenten aus thermoplastischem organischen Material zusammengesetzt ist.

8. Matte nach Anspruch 7, **dadurch gekennzeichnet, dass** die Filamente oder Fäden aus Glas, bevorzugt aus Glas E, sind.

9. Matte nach einem der Ansprüche 1 bis 8, **dadurch gekennzeichnet, dass** sie ein Flächengewicht von 10 bis 1100 g/m², bevorzugt von 20 bis 300 g/m², darstellt.

10. Matte nach einem der Ansprüche 1 bis 9, **dadurch gekennzeichnet, dass** die Fäden ungedreht sind.

11. Herstellungsverfahren der Matte nach einem der Ansprüche 1 bis 10, wobei eine Dispersion (11) aus geschnittenen Mineralfäden mittels eines Formkopfs (12) an einem mit Perforationen ausgestatteten Förderband (13) in Wasser gegeben wird, das Wasser mithilfe eines Saugkastens (14) extrahiert wird, die Fäden in Form einer Matte (15) gewonnen werden, ein Bindemittel (16) auf die Matte aufgebracht wird, die Matte durch eine Trocknungsvorrichtung (18) läuft und vom Band genommen wird, **dadurch gekennzeichnet, dass** es einen Bearbeitungsschritt mit einem Formaldehydfänger umfasst, der aus den vorgenannten aktiven Methylenverbindungen der Formel (I) bis (IV) ausgewählt wird.

12. Verfahren nach Anspruch 11, **dadurch gekennzeichnet, dass** die Bearbeitung darin besteht, den Formaldehydfänger in das Bindemittel (16) einzubringen.

13. Verfahren nach Anspruch 11, **dadurch gekennzeichnet, dass** die Bearbeitung darin besteht, den Formaldehydfänger nach Aufbringen des Bindemittels (16) auf die Matte und vor Einlauf der Matte in die Trocknungsvorrichtung (18) aufzutragen.

14. Verfahren nach Anspruch 13, **dadurch gekennzeichnet, dass** die Anwendung durch Aufsprühen einer wässrigen Lösung des Formaldehydfängers erfolgt.

## Claims

1. A mat of mineral yarns bonded with a binder, said yarns comprising a multitude of mineral filaments, and said yarn being cut to a length up to 100 mm **characterized in that** it includes an agent capable of trapping formaldehyde chosen from compounds comprising active methylene(s) corresponding to one of the following formulae (I) to (IV):
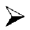 **FORMULA (I)** 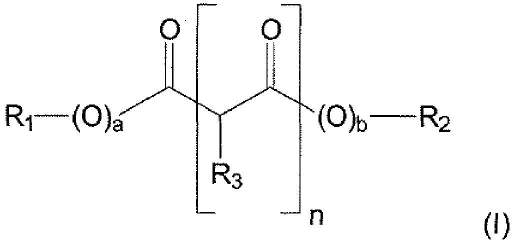 in which:
- R₁ and R₂, which are identical or different, represent a hydrogen atom, a C₁-C₂₀, preferably C₁-C₆, alkyl radical, an amino radical or a radical of formula 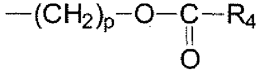 in which R₄ represents a 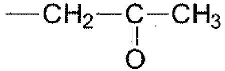 radical
or 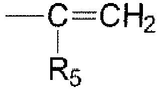 radical, where R₅ = H or -CH₃,
and p is an integer varying from 1 to 6,
- R₃ represents a hydrogen atom, a C₁-C₁₀ alkyl radical, a phenyl radical or a halogen atom,
- a is equal to 0 or 1,
- b is equal to 0 or 1,
- n is equal to 1 or 2,
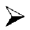 **FORMULA (II)**
R₆-CHR₇-C≡N (II)
in which:
- R₆ represents a cyano radical or a 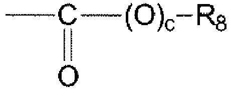 radical in which:
- R₈ represents a hydrogen atom, a C₁-C₂₀, preferably C₁-C₆, alkyl radical or an amino radical
- c is equal to 0 or 1
- R₇ represents a hydrogen atom, a C₁-C₁₀ alkyl radical, a phenyl radical or a halogen atom,
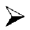 **FORMULA (III)** 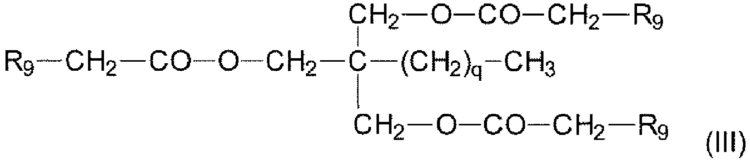 in which:
- R₉ represents a -C=N or -CO-CH₃ radical
- q is an integer varying from 1 to 4,
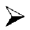 **FORMULA (IV)** 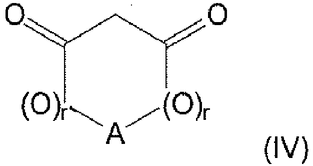 in which:
- A represents a -(CH₂)₃- or -C(CH₃)₂- radical
- r is equal to 0 or 1.

2. The mat as claimed in claim 1, **characterized in that** the compound of formula (I) is 2,4-pentanedione, 2,4-hexanedione, 3,5-heptanedione, 2,4-octanedione, acetoacetamide, acetoacetic acid, methyl acetoacetate, ethyl acetoacetate, n-propyl acetoacetate, isopropyl acetoacetate, isobutyl acetoacetate, t-butyl acetoacetate, n-hexyl acetoacetate, malonamide, malonic acid, dimethyl malonate, diethyl malonate, di(n-propyl) malonate, diisopropyl malonate, di(n-butyl) malonate, acetonedicarboxylic acid and dimethyl acetonedicarboxylate.

3. The mat as claimed in claim 1, **characterized in that** the compound of formula (II) is methyl 2-cyanoacetate, ethyl 2-cyanoacetate, n-propyl 2-cyanoacetate, isopropyl 2-cyanoacetate, n-butyl 2-cyanoacetate, isobutyl 2-cyanoacetate, tert-butyl 2-cyanoacetate, 2-cyanoacetamide and propanedinitrile.

4. The mat as claimed in claim 1, **characterized in that** the compound of formula (III) is trimethylolpropane triacetoacetate and trimethylolpropane tricyanoacetate.

5. The mat as claimed in claim 1, **characterized in that** the compound of formula (IV) is 1,3-cyclohexanedione and Meldrum's acid.

6. The mat as claimed in one of claims 1 to 5, **characterized in that** the content of compound capable of trapping formaldehyde varies from 0.1 to 500 g/m², preferably from 0.5 to 100 g/m² and advantageously from 1 to 50 g/m².

7. The mat as claimed in one of claims 1 to 6, **characterized in that** the mineral yarns are yarns composed of a multitude of mineral filaments (or base yarns) or assemblies of these base yarns in the form of rovings, "commingled" yarns composed of mineral filaments and of filaments of organic material which are intimately mixed, or mixed yarns comprising at least one yarn composed of a multitude of mineral filaments and at least one yarn composed of a multitude of filaments of thermoplastic organic material.

8. The mat as claimed in one of claims 1 to 7, **characterized in that** the filaments or the yarns are made of glass, preferably of glass E.

9. The mat as claimed in one of claims 1 to 8, **characterized in that** it exhibits a weight per unit area varying from 10 to 1100 g/m², preferably from 20 to 300 g/m².

10. The mat as claimed in one of claims 1 to 9, **characterized in that** the yarns are twist-free yarns.

11. A process for the manufacture of the mat as claimed in one of claims 1 to 10, in which a dispersion (11) of cut mineral yarns in water is deposited by means of a forming head (12) on a conveyor (13) provided with perforations, the water is extracted by means of a suction box (14), said yarns are collected in the form of a mat (15), a binder (16) is applied to said mat, said mat is passed into a drying device (18) and is collected, **characterized in that** it comprises a stage of treatment with an agent capable of trapping formaldehyde selected from compounds comprising active methylene(s) of formulae (I) to (IV) mentioned above.

12. The process as claimed in claim 11, **characterized in that** the treatment consists in introducing said agent in the binder (16).

13. The process as claimed in claim 11, **characterized in that** the treatment consists in applying said agent after the binder (16) is deposited on the mat and before the mat enters the drying device (18).

14. The process as claimed in claim 13, **characterized in that** the application is carried out by spraying an aqueous solution of said agent.
